# FASCICULE DE BREVET EUROPEEN

(11) **EP 1 097 130 B1**
(45) Date de publication et mention de la délivrance du brevet: **24.05.2006**
(21) Numéro de dépôt: 99929422.6
(22) Date de dépôt: 07.07.1999
(51) Int. Cl.: C07C 319/20, C07C 323/52

(54) **PROCEDE DE PREPARATION DE L'ACIDE HYDROXYMETHYLTHIOBUTYRIQUE**
VERFAHREN ZUR HERSTELLUNG VON HYDROXYMETHYLTHIOBUTTERSÄURE
METHOD FOR PREPARING HYDROXYMETHYLTHIOBUTYRIC ACID

(30) Priorité: 10.07.1998 FR 9808872
(43) Date de publication de la demande: 09.05.2001
(73) Titulaire: Adisseo Ireland Limited, Dublin 2 (IE)
(72) Inventeur: GARRAIT, Michel, F-69390 Millery (FR); GROS, Georges, F-92160 Antony (FR)
(74) Mandataire: Guerre, Dominique
(86) Numéro de dépôt international: PCT/FR1999/001637
(87) Numéro de publication internationale: WO 2000/002852

(56) Documents cités:
- US-A- 2 938 053
- US-A- 4 524 077
- US-A- 4 912 257

## Description

La présente invention concerne un nouveau procédé de préparation de l'acide 2-hydroxy 4-méthylthiobutyrique.

L'acide 2-hydroxy 4-méthylthiobutyrique est connu comme étant utilisé comme analogue de la méthionine pour nourrir les animaux d'élévage et principalement parmi ces animaux les volailles. Ce produit est commercialisé sous les marques Rhodlmet AT 88™ ou Alimet™.

Il est connu de préparer l'acide 2-hydroxy 4-méthylthiobutyrique par différents procédés d'hydrolyse du 2-hydroxy 4-méthylthlobutyronltrlle. L'hydrolyse est réalisée par un acide mlnéral tel que l'acide chlorhydrique ou sutftrrique ou peut encore être réalisée par hydrolyse enzymatique.

Il est connu selon le brevet GB No915 193 d'hydrolyser le 2-hydroxy 4-méthylthiobutyronitrile en acide 2-hydroxy 4-méthylthiobutyrique en présence d'un acide minéral. Ce brevet décrit l'hydrolyse continue avec un acide sulfurique dilué du 2-hydroxy 4-méthythiobutyrontrile et l'acide organique obtenu est récupéré par extraction avec un éther. Du au fait que l'hydrolyse est effectuée en continu dans un réacteur agité le procédé décrit dans ce brevet donne une hydrolyse incomplète du nitrile de départ et en conséquence la présence de dérivés indésirables qui ne peuvent être donnés aux animaux sans risque.

Il est aussi connu d'après le brevet US No4 524 077 d'hydrolyser le même nitrile de départ qui est le 2-hydroxy 4-mèthylthlobutyronitrile avec de l'acide sulfurique en deux étapes suivi d'une extraction du milieu d'hydrolyse par un solvant non miscible à l'eau. Le procédé en deux étapes consiste dans une première étape à utiliser un acide sulfurique ayant une concentration entre 50 et 70% en poids et à une température comprise entre 25 et 65°C. L'introduction du 2-hydroxy 4-méthylthiobutyronitrile est effectuée pendant une durée de 30 à 60 minutes et l'hydrolyse du nitrile en amide correspondant est effectuée pendant une période de 30 à 90 minutes. Le 2-hydroxy 4-méthylthiobutyramide est ensuite converti en acide 2-hydroxy 4-méthylthiobutyrique par une étape ultérieure d'hydrolyse à une température située dans une fourchette allant de 70 à 120 °C, L'étape finale d'hydrolyse est effectuée avec un acide ayant une teneur comprise entre 30 et 50% en poids. En pratique cette teneur est obtenue par addition d'eau. Dans ces conditions le 2-hydroxy 4-méthylthiobutyramide est transformé en acide 2-hydroxy 4-méthylthiobutyrique en 60 à 180 minutes. Pour transformer le nitrile en acide le rapport molaire de l'acide sulfurique par rapport au nitrile est compris entre 1 et 1.1.

Le brevet US No 4 912 257 décrit un procédé où le même nitrile c'est à dire le 2-hydroxy 4-méthylthiobutyronitrile est hydrolysé avec de l'acide sulfurique de telle façon que le rapport molaire de l'acide sulfurique avec le 2-hydroxy 4-méthytthiobutyronitnte soit compris entre 0.5 et 2 pour former un mélange réactionnel contenant 20-50% en poids d'acide sulfurique. Le mélange est maintenu à une tempéraure maximale de 50°C dans un réacteur agité pendant 30.60 minutes. La deuxième étape réactionnelle est réalisée dans un second réacteur chauffé à une température comprise entre 60 et 140°C pendant environ 5 à 6 heures.

La demande de brevet publiée sous le numéro WO 96/40630 décrit la même réaction d'hydrolyse en deux étapes. Le rapport préféré entre l'acide sulfurique et le 2-hydroxy 4-méthylthfobutyronitrile est toujours compris au départ de la mise en route de la réaction entre 1.15 et 1.25 puis lorsque la réaction est en état stationnaire entre 0.9 et 1.2 et plus préférentiellement entre 0.95 et 1.05. De nombreux exemples ont été réalisés pour faire varier ce rapport. Tous les exemples où le rapport est inférieur à 0.88 montrent un taux de conversion du 2-hydroxy 4-méthylthiobutyronitrile Inférieur à 95% ce qui est largement insuffisant pour une exploitatlon industrielle. Dans cette demande de brevet la conclusion est qu'il faut utiliser un rapport de l'acide sulfurique au nitrile compris entre 1.0 et 1.2.

L'inconvénient d'utiliser ces quantités d'acide sulfurique, comprises entre 1.0 et 1.2 mole par mole de 2-hydroxy 4-méthylthlobutyronitrlle est le fait qu'en fin de réaction on retrouve des quantités de sulfates d'ammonium proportionnelles aux quantités d'acide sulfurique introduites. Ces quantités élevées de sulfate provoquent un problème majeur de rejets industriels qu'il est de plus en plus difficile de résoudre. Par ailleurs le milieu étant fortement acide se trouve très corrosif à chaud et nécessite l'utilisation de matériaux exotiques. D'un point de vue réactionnel un rapport de 0.5 semblerait suffisant mais Il se révèle chimiquement dans les conditions utilisées jusqu'à maintenant inopérant. Il semblait donc impossible de descendre en ce qui concerne le rapport molaire de l'acide sulfurique par rapport au nitrile en dessous de 0.88 qui est l'extrême limite qui semble être opérable dans la demande de brevet cl-dessus évoquée.

Le document US-A-2,938,053 décrit un procédé de préparation de l'acide 2-hydroxy-4-méthylthiobutyrique (HMBA), en deux étapes à partir du 2-hydroxy-4-méthylthiobutyronitrile (HMBN).

Selon ce procédé :
On hydrate le HMBN en 2-hydroxy-4-méthylthiobutyramide (HMBM), dans les conditions suivantes : H₂SO₄/HMBN compris entre 0,5 et 0,8, température inférieure à 75°C ;
On hydrolyse le HMBM dans les conditions suivantes : dilution du milieu d'hydratation ci-dessus obtenu, dans de l'eau, à une température inférieure à 75°C ; élévation de la température à la température d'ébullition du milieu, à température atmosphérique.

Les rendements de l'hydratation de HMTBN en HMBM n'excèdent pas 75% par rapport au HMBN et ces conditions nécessitent un contrôle de la température de la réaction d'hydratation qui ne doit pas dépasser 75°C.

Il est apparu possible de réaliser l'hydrolyse du 2-hydroxy 4-méthylthiobutyronitrile en acide 2-hydroxy 4-méthylthlobutyrique avec d'excellents rendements avec un rapport molaire d'acide sulfurique par rapport au 2-hydroxy 4-méthylthlobutyronitrile compris entre 0.6 et 0.88. On préfère utiliser un rapport molaire compris entre 0.7 et 0,85.

La première étape qui est une réaction d'hydratation du 2-hydroxy 4-méthylthlobutyronitrile en 2-hydroxy 4-méthylthiobutyroamide est réalisée en milieu acide sulfurique très concentré et en présence d'une quantité d'eau suffisante pour réaliser cette réaction. La vitesse de cette réaction est inversement proportionnelle à la quantité d'eau. Ainsi une quantité d'eau variant entre une mole et trois moles d'eau par mole de 2-hydroxy 4-méthylthlobutyronitrile est nécessaire. On utilise encore plus préférentieiiement un rapport molaire entre l'eau et le 2-hydroxy 4-méthylthiabutyronitrile compris entre 1 et 2.5.

Cette faible concentration en eau limite très fortement lors de la première étape l'hydrolyse successive du 2-hydroxy 4-méthylthiobutyroamide en acide 2-hydroxy 4-méthylthiobutyrique. On préfère ainsi lors de cette première étape ne pas produire plus de 5%, de préférence moins de 2% en poids d'acide 2-hydroxy 4-méthylthiobutyrique. On préfère également lors de cette première étape obtenir une concentration en 2-hydroxy 4-méthylthiobutyroamlde supérieure à 95% en poids et de préférence supérieure à 98% en poids. Les conditions oprératoire utilisées lors de cette première étape sont choisies dans des limites ne conduisant pas à la production d'acide 2-hydroxy 4-méthylthiobutyrique, on travaille ainsi à une température inférieure ou égale à 60°C et notamment comprise entre 0°C et 50°C. La réaction est de préférence réalisée dans un système de réacteurs continu et en série avec un temps de séjour compris entre 15 minutes et 2 heures. La pression réactionnelle est de préférence choisie entre 0.01 et 3 bars.

La seconde étape de la réaction est une hydrolyse du 2-hydroxy 4-méthylthlobutyroamide en acide 2-hydroxy 4-méthyithiobutynque, elle est réalisée en présence de la quantité subsistante d'acide sulfurique non nsomm e dans la première étape et en présence d'une quantité d'eau supplémentaire évitant la séparation de phases dans le milieu réactionnel. Cette étape est de préférence réalisée en présence d'au moins 28% en poids d'eau. En ce qui concerne les conditions réactionnelles, on préfère travailler à une température comprise entre 90 et 130°C. De préférence on opère sous une pression comprise entre 0,5 bar et une pression de 5 bars. Une pression Inférieure à la pression atmosphérique permet d'éliminer des traces de gaz légers malodorant par exemple du type diméthylsulfure, diméthyldlaulfure et méthylmercaptan. Le faible excès d'acide et la présence de sulfate acide d'ammonium limitent fortement le pouvoir corrosif du milieu à cette température.

Selon le procédé de l'invention l'hydratation est réalisiée à partir d'une solution aqueuse de 2-hydroxy 4-méthylthiobutyronitrile qui est concentrée par évaporation de l'eau, au cours de la première étape. Lorsque l'on évapore l'eau contenue dans la solution aqueuse de 2-hydroxy 4-méthylthiobutyronitrile, l'eau évaporée à la première étape est avantageusement recyclée à la deuxième étape

Selon un meilleur moyen industriel de mise en oeuvre de l'invention on procède selon l'enchaînement des étapes suivant au départ d'une solution de 2-hydroxy 4-méthylthlobutyronitrile diluée :

Le 2-hydroxy 4-methytthiobutyronitrile concentré à environ 50 % en poids et l'acide sulfurique sont alimentés dans un appareil dans lequel on élimine une partie de l'eau apportée par les reactifs pour se ramener dans les conditions décrites précédemment et on hydrate le 2-hydroxy 4-methylthlobutyronitrile. On obtient ainsi une solution contenant du 2-hydroxy 4-methylthiobutyramide. On ajoute de l'eau, notamment l'eau éliminée précédemment, à cette solution avant d'hydrolyser le 2-hydroxy 4-methylthiobutyramide. La solution obtenue après hydrolyse contient de l'acide 2-hydroxy 4-methylthiobutyrique. L'acide 2-hydroxy 4-methylthiobutyrique est récupéré à partir de cette solution.

On peut, par exemple, réaliser ce procédé en continu, semi continu ou discontinu. Quand le procédé est réalisé en continu, l'appareillage utilisé pour l'hydratation du 2-hydroxy 4-methylthiobutyronitrile peut comprendre un premier réacteur agité fonctionnant sous faible pression. Les calories dégagées par la réaction servent à vaporiser l'eau excédentaire par rapport aux conditions du premier procédé de mise en oeuvre de l'Invention à partir d'une solution concentrée de 2-hydroxy 4-méthylthiobutyronitrile. On pourra terminer le procédé comme indiqué précédemment.

Selon un autre procédé de mise en oeuvre de l'invention on réalise la fin de la deuxième étape sous pression. L'hydrolyse du 2-hydroxy 4-mothylthlobutyramide accélère lorsque la température augmente. Pour dépasser la température d'ébullition du milieu on peut réaliser cette étape sous pression.

Le mélange obtenu est ensuite traité comme décrit dans les brevets US No4 524 077 ou US No 4 912 257. Ainsi le brevet US No 4 912 257 décrit à la suite de l'étape d'hydrolyse, une étape de neutralisation suivie par une étape de séparation biphasique et de séchage de chacune des deux phases suivie pour l'une des phases par une étape de filtration et pour l'autre par une étape de cristallisation. La mise au titre final se fait par addition d'eau.

Le brevet US No 4 524 077 consiste à réaliser une extraction directe évaporation dudit solvant en présence d'une quantité d'eau de façon à réduire l'apparition d'une coloration brune du produit obtenu. Le solvant est choisi notamment parmi la méthyléthylcétone, la méthylisobutylcétone, le méthyttertiobutytéther, le dilsopropyléther, le diéthylcarbonate.

Le procédé décrit dans le brevet US No 4 912 257 consiste à réaliser une séparation biphasique. On additionne au milieu issu de l'étape d'hydrolyse un agent de neutralisation du type ammoniaque. Le milieu se sépare en une phase organique (1) contenant l'acide recherché et des sels subslstants. La phase aqueuse (2) constituant l'autre phase contient essentiellement des sels minéraux, surtout du sulfate d'ammonium et des traces d'acide organique. Les deux phases peuvent être évaporées de façon à éliminer l'eau pour obtenir une solution organique d'acide 2-hydroxy 4-méthylthiobutyrique contenant de faibles quantités de.sulfate d'ammonium qui cristallise, ce dernier est séparé par filtration et l'acide 2-hydroxy 4-méthylthiobutyrique est adaptée au titre commercial recherché (88% en poids) par addition d'eau. Une autre solution consiste à se débarrasser des sels minéraux présents dans la solution d'acide 2-hydroxy 4-méthylihlobutyrique par addition d'un solvant organique peu miscible à Peau tel que notamment la méthyléthyl cétone, la méthyllsobutyl cétone, le diéthylcarbonate. On observe alors le relargage d'une phase aqueuse saline la phase organique est débarrassée du solvant par évaporation et la solution finale d'acide 2-hydroxy 4-méthylthiobùtytique est adaptée au titre commercial par addition d'eau.

La phase aqueuse (2) est évaporée de façon à précipiter les sels minéraux, essentiellement le sulfate d'ammonium qui peut être commercialisé tel quel mais qui contient des traces de dérivés organiques malodorants, Cette phase aqueuse peut aussi être traitée de façon à l'épuiser en acide 2-hydroxy 4-méthylthiobutyrique. Cet épuisement est réalisé par addition d'un solvant- peu miscible à l'eau choisi parmi la méthyléthyl cétone, la méthylisobutyl cétone et le diéthylcarbonate. La phase aqueuse débarrassée de ses dérivés organiques est séchée de façon à isoler les sels minéraux inodores et commercialisables directement La phase organique d'épuisement est recyclée par exemple avec la phase acide 2-hydro 4-méthylthlobutyrique afin de récupérer les quantités d'acide extraites de la phase aqueuse saline.

La présente invention sera plus complètement décrite à l'aide des exemples suivants qui ne doivent pas être considérés comme limitatifs de l'invention.

### EXEMPLE COMPARATIF1 Essai en réacteur fermé, à ratio H₂SO₄/Cyanhydrine =1.2

Dans un réacteur en verre de 250 ml muni :
- d'une double enveloppe dont la température est régulée par circulation d'huile,
- d'un agltateur,
- d'un condenseur.
- d'un thermocouple,
on charge un pied de 89 g de cyanhydrine à 78 % poids dans l'eau et 45 g d'eau. On obtient ainsi de la cyanhydrine à 52 % pds dans l'eau.
On ajoute progressivement 65.5 g d'acide sulfurique 95 % (le ratio molaire acide sulfurique/cyanhydrine est donc égal à 1.2, le ratio molaire eau/cyanhydrine est égal à 7.11) en maintenant la température du mélange réactionnel en dessous de 60 °C.
L'analyse du milieu montre que seulement 55 % de la cyanhydrine est convertie. Les sélectivités en HMTBM et en HMTBA sont, respectivement, de 91 % et 9 %.

On porte le mélange à ébullition, à 112 °C, pour terminer les réactions. Après 90 minutes dans ces conditions, l'analyse du milieu montre que :
- toute la cyanhydrine est convertie.
- les sélectivités en HMTBM et en HMTBA sont, respectivement, de 0.4 % et 99.6 %. Le ratio pondéral du sulfate d'ammonium produit par rapport à l'HMTBA est de 1.05.

### EXEMPLE COMPARATIF 2 Essai comparatif en réacteur fermé, à ratio H₂SO₄/Cyanhydrine=0.81

Dans un réacteur en verre muni :
- d'une double enveloppe dont la température est régulée par circulation d'huile.
- d'un agitateur,
- d'un condenseur,
- d'un thermocouple,
on charge un pied de 60 g de cyanhyddrie à 78 % poids dans l'eau et 60 g d'eau.
On ajoute progressivement 30 g d'acide sulfurique 95 % (le ratio molaire acide sulfurique / cyanhydrine est donc égal à 0.81 et le ratio molaire eau/cyanhydrine est égal à 44.27) en maintenant la température du mélange réactionnel en dessous de 60 °C.
Le milieu réactionnel étant hétérogène on ajoute 210 g d'eau pour obtenir un mélange homogène.
On maintient 30 minutes à 60 °C,
L'analyse du milieu montre que :
- 15 % de la cyanhydrine est convertie.
- les sélectivités en HMTBM et en HMTBA sont, respectivement, de 64 % et 36%.
On porte le mélange à ébullition, à 104 °C.
Après 160 minutes dans ces conditions, l'analyse du milieu montre que :
- seulement 29 % de la cyanhydrine est convertie.
- les sélectivités en HMTBM et en HMTBA sont respectivement, de 2% et 98 %. Le ratio pondéral du sulfate d'ammonium produit par rapport à l'HMTBA est de 0.72.

### EXEMPLE 1 Essai en réacteur continu sous vide (12 torr), à ratio H₂SO₄/Cyanhydrine=₀.₇₈

On alimente d'une part un mélange constitué par 163 g/h de cyanhydrine à 80 % et 62 g/h d'eau (ce qui correspond à 225 g/h d'HMTBN à 58 % poids) et d'autre part 80 g/h d'acide sulfurique à 95 %.
Le ratio molaire acide suffuriqueleyanhydrfne est donc égal à 0.78
La température du réacteur est maintenue à 50 °C. On fixe la pression à 12 torr.

Dans ces conditions on observe qu'en régime stationnaire
- on évapore 65 g/h d'eau, le ratio molaire eaulcyanhydrine est égal à 1.88.
- la conversion de la cyanhydrine est de 90 %. Les sélectivités en HMTBM et HMTBA sont de 98 % et 2%.

On coupe alors l'alimentation des réactifs, on coupe le vide, on maintient la régulation de température à 50 °C et on laisse évoluer le milieu réactionnel.
On suit la conversion de la cyenhydrlne :
- 2 minutes après l'arrêt la conversion de la cyanhydrine est de 99 %.
- 13 minutes après l'arrét la conversion de la cyanhydrina est de 100 %. Les sélectivités en HMTBM et HMTBA sont de 95 et 5%. Le ratio pondéral du sulfate d'ammonium produit par rapport à l'HMTBA est de 0.69.

On ajoute alors les 65g d'eau évaporée pendant la première partie de l'essai plus 39g d'eau supplémentaires et on porte le mélange à ébullition. à 110°C, pendant une heure. L'analyse du milieu montre que les sélectivités en HMTBM et HMTBA sont de 0.4% et 99.6%

### EXEMPLE 2 Essai en réacteur continu sous vide (12 torr), à ratio H₂SO₄/Cyanhydrine=0.8

On alimente d'une part un mélange constitué de 165 g/h de cyanhydrine à 80 % et 57 g/h d'eau et d'autre part 62 g/h d'acide sulfurique à 95 %.
Le ratio molaire acide sulfurique/cyanhydrine est donc égal à 0.6
La température du réacteur est maintenue à 50 °C. On fixe la pression à 12 torr.

Dans ces conditions on observe qu'en régime stationnaire :
- on évapore 62 g/h d'eau, le ratio molaire eau/cyanhydrine est égal à 1.71.
- la conversion de la cyanhydrine est de 80 %. Les sélectivités en HMTBM et HMTBA sont de 98 % et 2%.

On coupe alors l'alimentation des réactifs, on coupe le vide, on maintient la régulation de température à 50 °C et on laisse évoluer le milieu réactionnel.
On suit la conversion de la cyanhydrine :
- 5 minutes après l'arrêt la conversion de la cyanhydrine est de 98 %.
- 20 minutes après l'arrêt la conversion de la cyanhydrine est de 99.5 %. Les sélectivités en HMTBM et HMTBA sont de 95 et 5 %. Le ratio pondéral du sulfate d'ammonium produit par rapport à l'HMTBA est de 0.52.

On peut alors, ajouter de l'eau (par exemple l'eau évaporée dans cette étape) et porter le mélange à ébullition pour réaliser l'hydrolyse de l'HMTBM ainsi formé en HMTBA.

## Revendications

1. Procédé d'obtention de l'acide 2-hydroxy-4-méthylthÍobutyrique (HMBA) à partir du 2-hydroxy-4-méthyithiobutyronitrite (HMBN), ledit procédé comprenant :
l'hydratation du 2-hydroxy-4-m6thytthtobutyronitnte en 2-hydroxy-4-méthylthiobutyroamide (HMBM) par de l'acide sulfurique, en présence d'un rapport molaire de l'acide sulfurique au 2-hydroxy-4-méthytthiobutyronitrlle compris entre 0,6 et 0,88 et un rapport molaire de l'eau au 2-hydroxy-4-méthy)thiobutyronitrile compris entre 1 et 3, et
l'hydrolyse du 2-hydroxy-4-méthylthiobutyramide en acide 2-hydroxy-4-méthytthiobutyrique en présence d'une quantité supplémentaire d'eau,
**caractérisé en ce que** l'hydratation est réalisée à partir d'une solution aqueuse de 2-hydroxy-4-méthylthiobutyronitrile qui est concentrée par évaporation de l'eau, *au cours de la première étape,* et la température du milieu réactionnel maintenue Inférieure ou égale à 60°C.

2. Procédé selon la revendication 1 **caractérisé en ce qu'**à l'étape d'hydratation, le rapport molaire de l'acide sulfurique au 2-hydroxy-4-méthylthiobutyronitrile est compris entre 0,7 et 0,85.

3. Procédé selon la revendication 1, **caractérisé en ce qu'**à l'étape d'hydratation, le rapport molaire de l'eau au 2-hydroxy-4-méthylthiobutyronitrile est compris entre 1 et 2,5.

4. Procédé selon la revendication 1, **caractérisé en ce que** l'étape d'hydratation est réalisée sous une pression comprise entre 0,01 et 3 bar,

5. Procédé selon la revendication 1, **caractérisé en ce que** lors de la deuxième étape on ajoute une quantité d'eau suffisante pour maintenir le milieu sous une forme homogène.

6. Procédé selon la revendication 5, **caractérisé en ce que** la quantité minimale d'eau lors de la deuxième étape est de 28% en poids par rapport à l'ensemble du milieu réactionnel.

7. Procédé selon l'une quelconque des revendications 1 à 6, **caractérisé en ce que** l'eau évaporée lors de la première étape est recyclée à la deuxième étape.

8. Procédé selon la revendication 1, **caractérisé en ce que** la deuxième étape est réalisée à une température comprise entre 90 et 130°C.

9. Procédé selon la revendication 1, **caractérisé en ce que** la deuxième étape est réalisée sous une pression comprise entre 0,5 bar et 5 bar.

## Claims

1. Process for producing 2-hydroxy-4-methylthiobutyric acid (HMBA) from 2-hydroxy-4-methylthjobutyronitrile (HMBN), the said process comprising:
the hydration of 2-hydroxy-4-methylthiobutyronitrile to 2-hydroxy-4-methylthiobutyroamide (HMBM) with sulphuric acid, in the presence of a sulphuric acid to 2-hydroxy-4-methylthiobutyro nitrile molar ratio of between 0.6 and 0.88 and a water to 2-hydroxy-4-methylthiobutyronitrile molar ratio of between 1 and 3, and
the hydrolysis of 2-hydroxy-4-methylthiobutyrarnide to 2-hydroxy-4-methylthiobutyric acid in the
presence of an additional quantity of water,
**characterized in that** the hydration is carried ouL starting with an aqueous solution of 2-hydroxy-4-methylthiobutyronitrile which is concentrated by evaporation of the water, during the first, step, and the temperature of the reaction medium is kept less than or equal to 60°C.

2. Process according to Claim 1, **characterized in that** in the hydration step, the sulphuric acid to 2-hydroxy-4-methylthiobutyronitrile molar ratio is between 0.7 and 0-85.

3. Process according to Claim 1, **characterized in that** in the hydration step, the water to 2-hydroxy-4-methylthiobutyronitrile molar ratio is between 1 and 2.5.

4. Process according to Claim 1, **characterized in that** the hydration step is carried out at a pressure of between 0.01 and 3 bar.

5. Process according to Claim 1, **characterized in that** during the second step, a sufficient quantity of water is added in order to maintain the medium in a homogeneous torm.

6. Process according to Claim 5, **characterized in that** the minimum quantity of water during the second step is 28% by weight relative to the whole reaction medium.

7. process according to any one of Claims 7. to 6, **characterized in that** the water evaporated during the first step is recycled to the second step.

8. Process according to Claim 1, **characterized in that** the second step is carried out at a temperature of between 90 and 130°C.

9. Process according to Claim 1, **characterized in that** the second step is carried out at a pressure of between 0.5 bar and 5 bar.

## Patentansprüche

1. Verfahren zur Herstellung von 2-Hydroxy-4-methylthiobuttersäure (HMBA) aus 2-Hydroxy-4-methylthiobutyronitril (HMBN), das die folgenden Schritte umfaßt:
Hydratisierung von 2-Hydroxy-4-methylthiobutyronitril mit Schwefelsäure zu 2-Hydroxy-4-methylthiobutyramid (HMHM) bei einem Schwefelsäure/2-Hydroxy-4-methylthiobutyronitril-Molverhälthis zwischen 0,6 und 0, 88 und einem Wasser/2-Hydxoxy-4-methylthiobutyronitril-Molverhältnis zwischen 1. und 3 und
Hydrolyse von 2-Hydroxy-4-methylthioYautyramid zu 2-Hydroxy-4-methylthiobuttersäure in Gegenwart einer zusätzlichen Menge Wasser,
**dadurch gekennzeichnet, daß** man die Hydratisierung an einer wäßrigen Lösung von 2-Hydroxy-4 methylthiobutyronitril, die im Lauf des ersten Schritts durch Verdampfung von Wasser aufkonzentriert wird, durchführt und die Temperatur des Reaktionsmediums kleiner gleich 60°C hält.

2. Verfahren nach Anspruch 1, **dadurch gekennzeichnet, daß** im Hydratisierungsschritt das Schwefelsäure/2-14ydroxy-4-methylthiobutyronitril-Molvc,rliäl.tiiis zwischen 0,7 und 0,85 liegt.

3. Verfahren nach Anspruch 1, **dadurch** gekennzcichnet, daß im Hydratisierungssohritt das Wasser/2-Hydroxy-4-methylthiobutyronitril Molverhältnis zwischen 1 und 2,5 liegt.

4. Verfahren nach Anspruch 1, **dadurch gekennzeichnet, daß** man den Hydratisierungsschritt unter einem Druck zwischen 0,01 und 3 bar durchführt.

5. Verfahren nach Anspruch 1, **dadurch gekennzeichnet, daß** man im zweiten Schritt eine Menge Wasser zugibt, die ausreicht, um das Medium in homogener Form zu halten.

6. Verfahren nach Anspruch 5, **dadurch gekennzeichnet, daß** die Mindestmenge an Wasser im zweiten Schritt 28 Gew.-%, bezogen auf das gesamte Reaktionsmedium, beträgt.

7. Verfahren nach einem der Ansprüche 1 bis 6, **dadurch gekennzeichnet, daß** man das im ersten Schritt verdampfte Wasser im zweiten Schritt rezykliert.

8. Verfahren nach Anspruch 1, **dadurch gekennzeichnet, daß** man den zweiten Schritt bei einer Temperatur zwischen 90 und 130°C durchführt.

9. Verfahren nach Anspruch 1, **dadurch gekennzeichnet, daß** man den ersten Schritt unter einem Druck zwischen 0,5 und 5 bar durchführt.
